(19) Europäisches Patentamt / European Patent Office / Office européen des brevets

(11) **EP 4 327 790 A1**

(12) **EUROPEAN PATENT APPLICATION**

(43) Date of publication:
**28.02.2024 Bulletin 2024/09**

(21) Application number: **22192219.8**

(22) Date of filing: **25.08.2022**

(51) International Patent Classification (IPC):
**A61F 13/00** (2024.01)   **A61F 13/15** (2006.01)
**A61F 13/511** (2006.01)   **A61F 13/513** (2006.01)
**A61F 13/514** (2006.01)

(52) Cooperative Patent Classification (CPC):
**A61F 13/01008; A61F 13/01034; A61F 13/15252;
A61F 13/51121; A61F 13/513; A61F 13/51401;
A61F 13/5513**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Corman SpA
20084 Lacchiarella (IT)**

(72) Inventors:
• **MANTOVANI, Giorgio
20084 LACCHIARELLA (IT)**
• **MASSAGLI, Matteo
20084 LACCHIARELLA (IT)**

(74) Representative: **Blodig, Wolfgang
Wächtershäuser & Hartz
Patentanwaltspartnerschaft mbB
Weinstrasse 8
80333 München (DE)**

(54) **BIODEGRADABLE ABSORBENT PRODUCT**

(57)    The invention describes an absorbent product having a sequence of layers and comprising at least the following layers in this order:
(i) a fluid-permeable top sheet facing the body of the user,
(ii) an acquisition and distribution layer (ADL),
(iii) an absorbent layer, and
(iv) a fluid-impermeable back sheet,
wherein at least layers (i) and (iv) are or comprise a sheet made of a polysaccharide.

Fig. 1

## Description

FIELD OF THE INVENTION

The present invention relates to an absorbent product comprising multiple layers, wherein layers of said product comprise one or more sheets made of polysaccharide, such as a cellulose material and/or material of a cellulose derivative. The absorbent product may be a wound dressing or a hygiene product like a diaper. The invention also provides a packaged absorbent product comprising the absorbent product.

BACKGROUND OF THE INVENTION

Disposable absorbent articles such as sanitary towels, pads, sanitary napkins, diapers (napkins) etc. are designed and offered for use as devices absorbing and storing body exudates like urine, menstrual blood, wound exudate, and faeces. In the history of product development following the user's desire to improve hygiene, comfort, ease of application and discretion, washable, textile products that had been in use for centuries were replaced by disposable single-use products.

These disposable products are generally laid-out and manufactured as layered products, typically comprising a liquid pervious top sheet, a liquid impervious back sheet bonded to said top sheet or connected at least at the periphery of the product to the top sheet, whereby these two layers are bonded together to form an "envelope" having an absorbent core positioned between said top sheet and said back sheet and in some cases an acquisition and distribution layer (ADL) positioned between said top sheet and said absorbent core.

Hygiene absorbent products made of materials consisting of or comprising cellulose fibers and films are known and were dominating in the markets of 1930s till 1950s, as in those times synthetic polymers were not yet available in sufficient amounts or were too expensive for applications in single-use disposable mass consumption products. Their performance and user's comfort were low compared to today's product standards, but they were more convenient than reusable, washable products dominating the markets earlier.

Examples of such products comprising layers of tissue paper, cotton fibers and/or cellulose film are known in patent literature. GB520576 A, published in 1940, describes a catamenial bandage made of cellulose absorbent material and wrapped in a "cellulose hydrate" foil. The wrapping is folded with an overlap and is provided with an easily torn off panel having a gripping-tongue so that the pad may be allowed to drop out of the wrapping.

GB373758 A, published in 1932, describes a catamenial pad (sanitary towel) which comprises absorbent material of cellulose wadding or absorbent cotton wadding, completely surrounded, except for a narrow strip on its upper side, by moisture repelling material, such as raw cotton, non-absorbent cottonwool, or wool. The pad, in use, is enclosed in a gauze envelope. GB549254 A, published in 1942, describes a catamenial bandage comprises a filling of absorbent material and an outer confining casing which is pervious to moisture and impervious to the filling and is made from a soft sheet of relatively long corded fibers strengthened by lines of a bonding medium, e.g., latex, spaced a distance less than the average length of the corded fibers. US2024976 A, published in 1935, describes an all-disposable sanitary napkin, including a filling, and sheeted cellulose wadding substantially surrounding the filling in conformable relation, said wadding being fluid repellant for substantially all that area surrounding one face and the side margins of the filling and a gauze casing. GB362046 A, published in 1932, relates to a sanitary napkin of the kind comprising several sheets of absorbent paper, a gauze wrapper, and cushioning material between the filling and the gauze for imparting softness. According to the invention, the cushioning material consists of layers or bats of absorbent. GB430269 A, published in 1935, describes a sanitary towel comprising a pad of absorbent material enclosed in a wrapper of open mesh textile material, and cushion strips interposed between the wrapper and the pad which are so arranged that each strip overlies a side edge and comparatively narrow marginal portions of one or both surfaces of the pad. The cushion strips are thin and are preferably formed of soft, loosely felted cotton fibers.

It is common to these product designs that they comprise layers of materials constituting and fulfilling the functions of an absorbent core, wrapping of this core and a casing of the entire product. Fluid management improving the flow, the distribution of the fluid within the core and reducing the re-wet are not considered. The choice of mostly cellulosic materials is a result of the availability of these materials in those times.

In past four decades, the functional layers of absorbent hygiene products as well as the product design have been further developed and their specific performance as well as the performance of the entire product comprising these functional layers reached a high level, resulting in high consumer appreciation of absorption speed, absorption capacity, fit, softness and wearing comfort, being non-obtrusive, dryness in use after wetting and safe disposal. In many cases, modern functional layers are made from at least two sub-layers providing different properties to these materials. Such modern state-of-the-art products are described in numerous patents, as an example in US 10,105,269, US 9,579,238 and US2019201252 A1.

Although cotton has been known in Europe since more than 2000 years, its use in wound treatment only begun in the 19th century. Before that, untreated cotton was used as an insulation for wounds, mechanically protecting a dressed wound from pressure and sometimes used as a wick to allow for a flow of wound exudates. Chemical cotton bleaching and application of disinfectants opened the way to the use of this material in wound treatment. A description of the history of cotton applications in the field of wound treatment is given in the article of J.R. Elliott "The Development of Cotton Wool as a Wound Dressing" (Med. Hist. 1957 Oct, 1(4): 362-366.)

The switch from cotton wool to both woven and nonwoven fabrics made of bleached cotton was a further step in the development of cotton wound dressings, as it allowed for reduction of problems with fibers sticking to or encased by wound exudates and scabs, which often resulted in pain when the dressings were removed from the wound.

While cotton gauze sponges still dominate the field of acute wound treatment in surgical applications, the modern long-term wound therapy rather uses other products providing a complex performance profile of non-sticking to the wound, keeping a balanced level of humidity, being anti-microbial and breathable.

Commonly, the wound dressings are divided into the group of "traditional dressings", made of cotton gauze, cotton nonwovens and sometimes of viscose and "modern dressings", made of hydrocolloids, hydrogels, alginates, polyesters, polyamides, polyurethanes, silicones, polyacrylates etc. Such modern wound dressings are described in many patent documents. EP0878204 A2 describes a superabsorbent wound dressing comprising superabsorbent fibers made of polyacrylnitrile. EP1435247 A1 describes a wound dressing having a top sheet (wound contact layer) made of gel-forming alginate fabric and an absorbent layer comprising superabsorbent material in fibrous form. EP0405993 describes a wound dressing formed by drying a water-swellable, waterinsoluble absorbent composition.

Even if considered "old style", cellulosic fibers have interesting properties and can be used - mostly in combination with hydrogels - in composite materials providing a good healing performance in modern wound therapies. Examples of such product innovations are described by E. Pinho and G. Soares in their publication "Functionalization of cotton cellulose for improved wound healing" (Journal of Materials Chemistry B, Issue 13, 2018).

Abazari et al. published in 2021 a review on "Recent advances in cellulose-based structures as the wound-healing biomaterials. A clinically oriented review" (Appl. Sci. 2021, 11, 7769). The authors describe the wound healing process and the role of wound dressings in it as well as wound healing challenges in the clinic. Following on this introduction, wound dressings made of biomaterials based on cellulose acetate, carboxymethyl cellulose and bacterial cellulose (also known as microbial cellulose) are described. For example, hydrogels made of cellulose acetate and of carboxymethyl cellulose can be successfully use in wound dressings. Bacterial cellulose is very similar to plant-extracted cellulose, but offers thinner fibrils which results in higher surface area.

Carboxymethyl cellulose (CMC) can be used as a superabsorbent polymer suitable for use in catamenial products as well as in wound dressings as CMC shows good absorption performance for blood, menstrual blood, and serous body fluids.

The Abazari publication is extremely valuable for product designers as it lists and characterizes a large number of cellulose-based substances which offers a perfect source for educated choice of the right ones in a specific hygiene product or wound dressing. The material lists include 21 different cellulose acetate-based materials, 22 CMC-based materials and 18 bacterial cellulose-based materials. For those wishing to deeper dig in the details about these materials, the publication offers 151 literature references.

One of important steps in wound healing is the wound cleansing phase which can be supported by a proper wound dressing. Wild et al. (Wound cleansing efficacy of two cellulose-based dressings, Wounds UK, 2010, Vol. 6. No.3) report a good cleansing and debridement properties of both tested cellulose-based dressings.

Carboxymethyl cellulose (CMC) was described in the cited Abazari publication. Another paper "Carboxymethyl cellulose-based materials for infection control and wound healing: A review" published in International Journal of Biological Macromolecules (Volume 164, 1 December 2020, pages 963-975) highlights interesting properties of CMC like its biocompatibility, biodegradability, tissue reassembling at relatively low cost.

In modern regenerative medicine, cellulose is recognized as a material actively supporting tissue regeneration. "Cellulose - a biomaterial with cell-guiding property", a publication of Tomilla et al. (https://www.intechopen.com/chapters/45634) describes successful experiments with cellulose-based tissue scaffolds supporting tissue regeneration.

Even simple cellulose fibers like cotton or woodpulp fibers have advantageous properties supporting wound healing. It is known that they attract cell debris contained in wound exudates which directly supports wound cleansing and healing. Specifically, if wet, cellulosic fibers as those of cotton can filter out the cell debris and other organic compounds out of the wound exudate and thus keep them away from the wound as well as reduce the risk of their further decomposition often resulting in creation of toxins.

The introduction of superabsorbents to hygiene products in early 1980s opened the way for development of modern wound dressings comprising superabsorbent polymers and offered advantages in an advanced wet wound therapy. Such products were soaked with aqueous solutions of some electrolytes (e.g., so called Ringer-Solution) and positioned on the chronic al wound for at least one day. The superabsorbent particles within the dressing helped to keep the wet environment for longer time and supported the removal of cell debris and other proteins from wound exudates as superabsorbents show similar affinity to proteins as cellulose fibers. An example of such product is described in a US6169223 issued to Mahr et al. in 2001. Products described in this patent were successfully offered to the market under the brand of "Tenderwet" changed later on to "Hydro Clean". From the sustainability point of view, these products show a disadvantage as their outer cover is made from synthetic polymers like polypropylene (PP) and an internal layer is made of polyethylene (PE), thus plastics.

Artificial fossil-fuel based polymers such as PP or PE consume valuable fossil fuels and generate carbon dioxide when products are thermally utilized, i. e. incinerated, after use in waste removal procedures. This is particularly problematic, since such products are single-use disposable products that are utilized in huge numbers world-wide.

Departing from the prior art, it is a technical problem of the invention to provide an absorbent product of good fluid management and comfort in use, while being at the same time of high or improved sustainability and environmental friendliness.

SUMMARY OF THE INVENTION

For solving this problem, the invention provides:

1) An absorbent product having a sequence of layers and comprising at least the following layers in this order:

(i) a fluid-permeable top sheet facing the body of the user,
(ii) an acquisition and distribution layer (ADL),
(iii) an absorbent layer, and
(iv) a fluid-impermeable back sheet,

wherein at least layers (i) and (iv) are or comprise a sheet made of a polysaccharide, preferably made of cellulose or a cellulose derivative.

2) The absorbent product according to item 1), wherein the median pores size of pores in the top sheet is at least 20 times, preferably at least 50 times, more preferably at least 100 times larger than the median pore side of pores in the back sheet.

3) The absorbent product according to items 1) or 2), wherein layers (i), (ii), and (iv) comprise one or more sheets made of cellulose or a cellulose derivative.

4) The absorbent product according to any one of items 1) to 3), wherein each of said four layers (i) to (iv) comprises one or more sheets consisting of or made of a cellulose material or of a material of a cellulose derivative as the material forming said sheets, preferably of cellulose.

5) The absorbent product according to any one of items 1) to 4), wherein said top sheet is or comprises a sheet that is a fabric made of cellulose fibers, such as of cotton and/or viscose fibers, preferably said fabric is a nonwoven fabric that may be produced by hydroentanglement (spunlacing).

6) The absorbent product according to item 5), wherein said cellulose or viscose fibers used for hydroentanglement have a median length of 8 to 30 mm, preferably from 10 to 20 mm.

7) The absorbent product according to item 5) or 6), wherein said top sheet is at least partially coated with a hydrophilic polymer, such as carboxymethyl cellulose, alginate or hydrogels, on its outer surface facing the body of the user.

8) The absorbent product according to any one of items 1) to 7), wherein said top sheet has perforations for improved transfer of body exudate to the ADL and absorbent layer.

9) The absorbent product according to any one of items 1) to 8), wherein said ADL is or comprises a fibrous batt, preferably a nonwoven fabric made of cellulose or a cellulose derivative.

10) The absorbent product according to item 9), wherein said ADL is or comprises a nonwoven fabric made of cellulose fibers and/or of fibers of a cellulose derivative, preferably made of cross-linked cellulose fibers, said fabric preferably having a density of 0,20 g/cm$^3$ or lower.

11) The absorbent product according to item 10), wherein said fibers have, before cross-linking, a median length of 2 mm or longer, preferably a median length of from 2 to 15 mm.

12) The absorbent product according to any one of items 9) to 11), wherein said nonwoven fabric has a wicking rate with synthetic urine at least two times higher than the wicking rate of the top layer.

13) The absorbent product according to any one of items 1) to 12), wherein said absorbent layer comprises a superabsorbent, preferably the superabsorbent comprises or consists of a cellulose derivative.

14) The absorbent product according to any one of items 1) to 12), wherein said absorbent layer comprises one or more sheets and a superabsorbent within said one or more sheets, said sheets being made of cellulose and/or of a cellulose derivative.

15) The absorbent product according to any one of items 1) to 14), wherein said absorbent layer comprises two or more sheets enclosing a superabsorbent between said sheets, said sheets being made of cellulose and/or of a cellulose derivative, such as cellulose fibers.

16) The absorbent product according to item 14) or 15), said one or more sheets being made of dry defiberized cellulose, such as fluff, and/or being made of cellulose tissue paper.

17) The absorbent product according to any one of items 14) to 16), wherein said one or more sheets is/are obtainable from dry defiberized cellulose pulp by laying down the pulp by an air stream and subsequently compressing to a density of at least 100 kg/m$^3$.

18) The absorbent product according to any one of items 13) to 17), wherein said absorbent layer has, at first wetting, a wicking rate with synthetic urine of at least two times the wicking rate of the top layer.

19) The absorbent product according to any one of items 13) to 18), said absorbent layer comprising a nonwoven fabric sheet made of cellulose fibers and/or of fibers of a cellulose derivative, and particles of a superabsorbent attached to said nonwoven fabric sheet, said superabsorbent preferably comprising or consisting of a cellulose derivative.

20) The absorbent product according to any one of items 1) to 18), said absorbent layer comprising at least two sheets of porous cellulose material, such as tissue paper or wet-laid or spun-laced nonwoven, and superabsorbent polymer particles between said layers.

21) The absorbent product according to any one of items 13) to 20), wherein said superabsorbent is or comprises carboxymethyl cellulose (CMC) and/or a polyacrylic acid, or salts thereof, preferably said superabsorbent consists of CMC or a salt thereof, more preferably particles of CMC.

22) The absorbent product according to any one of items 1) to 21), wherein said back sheet is or comprises a sheet or film of cellulose material or material of a cellulose derivative, preferably said back sheet is a cellulose film such as a cellophane film; said back sheet may contain or may be coated with a softening agent such as glycerol.

23) The absorbent product according to any one of items 1) to 22), wherein said back sheet comprises or consists of a layer of a cellulose film or a film of a cellulose derivative laminated with layer of a nonwoven made of cellulose fibers, wherein said film laminated with a nonwoven preferably has a basis weight of 5 to 40 g/m$^2$, preferably 10 to 30 g/m$^2$, and even more preferably 15 to 25 g/m$^2$.

24) The absorbent product according to any one of items 1) to 23), wherein said back sheet comprises cellulose fibrous material, said sheet having pores with a pore size not large enough for water droplet transmission but large enough for water vapor transmission, thus being breathable; preferably, the pore size of said sheet being smaller than 100 $\mu$m, preferably smaller than 50 $\mu$m.

25) The absorbent product according to any of items 1) to 24) wherein adjacent layers and/or sheets can be bonded to each other and form a composite material.

26) The absorbent product according to any one of items 1) to 25), wherein the top sheet and the back sheet are bound to each other by mechanical embossing, preferably at least along the periphery of said product so that the product forms a bag-like structure enclosing the absorbent layer and optionally the ADL.

27) The absorbent product according to any one of items 1) to 26), which is an absorbent hygiene product such as a sanitary napkin, a napkin, or an incontinence pad, or is a wound dressing.

28) An absorbent product having a sequence of layers and comprising at least the following layers in this order:

   (i) a fluid-permeable top sheet facing the body of the user, said top sheet being made of a polysaccharide, preferably made of cellulose or a cellulose derivative,
   (ii) an acquisition and distribution layer (ADL) comprising or consisting of a sheet made of cross-linked cellulose, preferably a nonwoven made of cross-linked cellulose,
   (iii) an absorbent layer comprising a superabsorbent consisting of a cellulose derivative, preferably carboxymethyl cellulose, and optionally a sheet made of cellulose or a cellulose derivative, and
   (iv) a fluid-impermeable back sheet made of a polysaccharide.

29) The absorbent product according to any one of items 1) to 28), which does not contain a non-biodegradable polymer in an amount of more than 3 % by weight, or does not contain polyethylene and/or polypropylene in a combined amount of more than 3 % by weight of said product.

30) A packaged absorbent product comprising the absorbent product according to any one of items 1) to 29) wrapped in a film made of cellulose material or material of a cellulose derivative, such as cellophane, or wrapped in a sheet made of cellulose fibers.

The inventors have studied various materials that may be used for producing biobased and biodegradable absorbent products. The inventors have surprisingly found that it is possible to make a multi-layered absorbent product that is functionally equivalent for users to state-of-the-art products made of raw oil. The invention provides products such as absorbent hygiene products and wound dressings, wherein sheets are made of a polysaccharide, preferably cellulose or a cellulose derivative. Cellulose can inter alia be obtained from natural sources such as cotton, i. e. from renewable sources. Since polysaccharides such as cellulose and cellulose derivatives are biodegradable, absorbent products can be disposed of as part of organic waste and even be used for biogas production in biogas production facilities, e. g. together with agricultural waste. Even if disposed waste is incinerated, the carbon dioxide produced will be largely equivalent to the amount of carbon dioxide bound by carbon fixation from atmospheric carbon dioxide (e. g. by photosynthesis). Thus, the absorbent product of the invention is of excellent environmental friendliness and sustainability.

BRIEF DESCRIPTION OF THE DRAWINGS

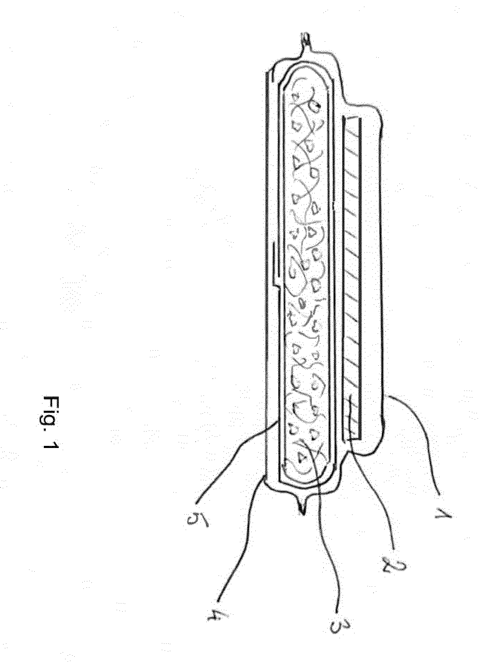

Fig. 1 shows schematically a cross-section through an absorbent product according to the invention. Numeral 1 indicates the top sheet of the product. Numeral 2 identifies an ADL of the product. Numeral 3 indicates the absorbent core of the product. The absorbent core is enveloped by the sheet identified by numeral 5. The absorbent core 3 and the envelope sheet 5 together form the absorbent layer. Numeral 4 identifies the back sheet of the product. Extensions of the top sheet and of the back sheet form the boundaries of the product also laterally and are bonded to each other, so as to enclose the ADL and the absorbent layer.

DETAILED DESCRIPTION OF THE INVENTION

The absorbent product of the invention comprises at least four layers (i) to (iv) that provide, respectively, the main functions of (i) permeation of fluid released from the body of a user through the top sheet, (ii) acquisition and distribution of the fluid transmitted through the top sheet, (iii) absorbing and retaining the body fluid, and (iv) preventing release (leakage) of fluid from the product on the side facing the outside (i.e. side opposite to that facing the body of the user). Generally, the top sheet and the back sheet further function as outer physical boundary of the product, that encloses the ADL and the absorbent layer. Each of layers (i) to (iv) may comprise or consists of more than one layer. For example, the top sheet may be two-layered sheet.

The product generally has a flat shape with two long dimensions (x- and y-direction in the plane of the flat shape) and one short dimension (z-direction) that defines the thickness of the product. The product is, however, flexible to be able to adjust to the body shape of the user. At least layers (i) and (iv) are or comprise a sheet. Preferably, each of elements (i), (ii) and (iv) are or comprise a sheet. In one embodiment, also element (iii) comprises a sheet. In the invention, the sheets are made of (preferably consist of) a polysaccharide. The polysaccharide is preferably cellulose and/or a cellulose derivative.

Herein, an element referred to as "sheet" extends essentially over the entire surface of the flat product and has stability and integrity to provide, together with other sheets, the physical integrity of the product. A sheet contributes to the overall strength of the absorbent product. Thus, a sheet has mechanical strength and integrity sufficient so that the sheet can be handled without destruction in the production process of the product. A sheet of the invention is flexible allowing bending so that the absorbent product is also flexible and can adapt to the body shape of the user. The sheets are elements that, due to their size (extension), strength and integrity pose the main problem for degradation of the product after use, particularly in the absence of sustainable waste treatment. Therefore, in the invention, the sheets are made of polysaccharide in order to be biodegradable, preferably are made of cellulose material and/or material of cellulose derivatives.

Being made of polysaccharide means that the structural components that provide the sheet with the strength, integrity and stability are (preferably consist of) polysaccharide(s), preferably cellulose and/or a cellulose derivative. However, a sheet may additionally comprise further materials or components, e.g. for modifying the surface properties of the sheet. In one embodiment, a sheet may be coated with a material that is not polysaccharide. The coating may be with biodegradable hydrophilic polymers (see further below). In another embodiment, a sheet may contain or be coated with a softening agent, such as glycerol. A sheet of the invention should not contain particles (such as fibers of flakes) of non-biodegradable material, notably not of organic polymers, in particular not of PE and PP. More preferably, a sheet of the invention should not contain non-biodegradable material, notably not organic polymers, in particular not PE and PP. A sheet may, however, contain particles of inorganic materials, such as mineral powders (e.g. talc, magnesia, silica). Examples of sheets are foils, films, paper webs and nonwovens.

Most preferably, all sheets of the product of the invention are of made of polysaccharide, notably of cellulose material and/or material of cellulose derivatives. In a preferred embodiment, all layers (i) to (iv) consist of biodegradable materials, preferably of polysaccharides.

Herein, biodegradable material is material that degrades in nature by the action of organism, notably by microbial attack. Biodegradability is of particular importance for polymeric material, since they frequently enter the environment as particulate material. The monomer units of biodegradable polymeric material are generally linked by hydrolysable bonds, such as ester or ether bonds, notably glycosidic ether bonds of polysaccharides. Examples of biodegradable polymeric materials are proteins and polysaccharides, but also polyethylene glycol (PEG). Polysaccharides (also called glycans) are compounds consisting of a large number of monosaccharide units linked by glycosidic bonds (i.e. glycosidically). Examples of polysaccharides are cellulose and its derivatives, starch, glycogen, pectin, alginate or a salt thereof, and hyaluronic acid or a salt thereof. Biodegradable material may be natural products, notably plant products, containing biodegradable polymeric material. A natural product of high utility in the present invention is cotton. Cotton is a staple fiber that is almost pure cellulose, but may contain minor amounts of other components, such as waxes, fats, and pectins. The latter are also biodegradable. For the purposes of this invention, small-molecular compounds (molecular weight smaller than 500 Da) and inorganic compounds are considered biodegradable. For example, a product containing talc or silica on a sheet, e.g. on the top sheet, but otherwise consisting of polysaccharide, is considered biodegradable.

The sheets used in the invention preferably do not contain a non-biodegradable polymer (irrespective of being particulate or not). Herein, non-biodegradable polymers are polymers the monomer units of which are linked by carbon-carbon bonds. Examples of such polymers are polyethylene (PE) and polypropylene (PP). Another example non-biodegradable polymer is PET (polyethylene terephthalate). Most currently used hygiene products in the market contain one or more of these non-biodegradable polymers. The absorbent product of the invention does preferably not contain a non-biodegradable polymer in an amount of more than 3 % by weight, preferably of more than 1 % by weight of the entire product, preferably of not more than 0.5 % by weight. In one embodiment, the absorbent product does not contain PE nor PP nor PET in an amount of more than 3 % by weight, preferably not more than 1 % by weight of the entire product, preferably not more than 0.5 % by weight, whereby these values apply to the combined content of any PE, PP, and PET.

Herein, a polysaccharide (or glycan) is a compound consisting of a large number of monosaccharide units linked glycosidically (IUPAC Gold Book). This term is commonly used only for those containing more than ten monosaccharide residues. A monosaccharide is an aldose, ketose or a derivative of hydroxy groups thereof, selected from oxidation, deoxygenation, replacing hydroxy groups by other substituents (e.g. amino or N-acetylamino), alkylation and acylation of hydroxy groups.

Cellulose is a polysaccharide consisting of D-glucose units linked by $\beta$-1,4-glycosidic bonds. In cellulose derivatives, hydroxy groups of D-glucose units (other than those involved in $\beta$-1,4-glycosidic bonds) may be chemically modified. Examples of modifications are alkylation (such as methylation), carboxymethylation, acylation (such as acetylation, to form cellulose esters), and nitration. The corresponding cellulose derivatives are alkylcellulose (such as methylcellulose or ethylcellulose), carboxymethyl cellulose (CMC), cellulose acetate, and nitrocellulose (cellulose nitrate), respectively. Herein, the alkyl group is a $C_{1-4}$ alkyl group. A further possible cellulose derivative is cross-linked cellulose. Methods of producing such cellulose derivatives from cellulose are well-known in the art. Preferred cellulose derivatives are CMC (notably as absorbent) and cross-linked cellulose. The cross-linking of cross-linked cellulose should be such that biodegradability is not prevented, e.g. by citric acid.

Cellulose and cellulose derivatives, by way of the essentially linear structure of its polymer molecules, is well suitable for forming macroscopic particles or objects of mechanical strength, such as natural or artificial fibers, yarns made from the fibers, as well as flat objects such as sheets. Examples of sheets are paper sheets, cellophane sheets, nonwoven fabrics, and fiber-containing essentially flat (planar) sheets. Herein, the terms "nonwoven" and "nonwoven fabric" are used interchangeably. The sheets of the invention are preferably made of cellulose or cellulose derivatives.

Preferably, all sheets of the product are made of cellulose and/or a cellulose derivative, cellulose being preferred. Layer (iii) does not necessarily contain a sheet. However, also layer (iii) may comprise a sheet made of cellulose or a cellulose derivative, preferably cellulose. Thus, in one embodiment, each layer (i) to (iv) comprises at least one sheet that is made of cellulose or a cellulose derivative, cellulose being preferred.

Cellulose is a material class comprising many materials that may differ in many respects. On the molecular level, cellulose and cellulose derivatives may differ in molecular weight and molecular weight distribution of the cellulose molecules contained, and/or degree of crystallization. On the macroscopic level, cellulose materials and materials of cellulose derivatives may take different forms, such as paper, fibers, yarn made from fibers, woven fabrics made from yarn, nonwoven fabrics etc. The terms "cellulose materials" and "materials of cellulose derivatives" thus relate to the macroscopic manifestation of cellulose and cellulose derivatives, respectively, such as bulk material. Fibers may differ in fiber size (thickness and length and the distribution of thickness and length), as well as in procedures available to form fibers and fabrics. Examples of different cellulose materials are as follows:

fibers: long, short, thin, thick, natural, regenerated, crosslinked, curled;
fabrics made of these fibers: hydrophilic, hydrophobic, thick, thin, with different fiber orientation (e.g. in z-direction), voluminous, flat, apertured;
films made of regenerated cellulose: cellophane and similar materials, opaque, transparent, calandered, softened, breathable;
papers made of cellulose fibers: tissue, tissue wrapping corrugated papers;
paper-like wet-laid nonwovens: hydrophilic, hydrophobic, breathable;
superabsorbents: CMC.

For the sheets used in the invention, nonwoven fabrics (nonwovens) may be used. By ISO and CEN, nonwovens are defined as engineered fibrous assembly, primarily planar, which has been given a designed level of structural integrity by physical and/or chemical means, excluding weaving, knitting or paper making. Nonwovens used to make sheets for the absorbent products of the invention may be carded or spun bonded, made of cellulose materials. Nonwovens made of natural or regenerated natural fibers like cotton or viscose, can be manufactured by using spun-lace hydroentanglement technology.

The different layers of the absorbent product of the invention generally differ in physical requirements dependent on the respective functions of the layers. Thus, generally, different polysaccharides are used for these layers in order to achieve the various functions of the layers. In particular, different types of cellulose materials or materials of cellulose derivatives may be used for the layers of the product. These aspects will be described in the following for the layers of the product.

Top sheet

The top sheet should be soft and gentle as it contacts the skin of the user. Moreover, it should allow fast acquisition of fluid from the body of the user and quickly passing the fluid on to the inner layers of the absorbent product. The top sheet should remain dry after passing the fluid to the inner layers. Top sheets of modern absorbent products are generally made of fibers consisting of, typically, a polypropylene core and polyethylene sheath, carded or spunbonded and air-trough bonded. Such top sheet nonwovens are further described in EP 91905693 B1, US3489148 A, US8530722, or EP2399558. A new generation of top sheets made by air-through bonding technology provides a significant improvement towards even softer, bulkier, and skin-friendly nonwovens. Examples of such materials are described in EP1369519, in EP 2 708 623 and WO2014022988. A method for producing such high loft nonwovens is described in EP3246444. While information on physical properties of top sheets may be taken from these publications, such top sheets are not or only partially biodegradable and are therefore not suitable for the product of the invention.

The top sheet of the product of the invention is fluid permeable. In the invention, the fluid may be water or a body exudate such as blood. Fluid permeability may be determined using water.

The top sheet preferably is or comprises a sheet that is a fabric made of fibers of cellulose, e.g. cotton or viscose fibers, or of fibers of a cellulose derivative, e.g. fibers made of cellulose esters such as described in DE3312022 A1 and DE3246417 A1. Preferably, the fabric is a nonwoven fabric made of cellulose fibers. The nonwoven may be produced by hydroentanglement from fibers (also referred to in the art as "spunlacing"). The fibers (such as viscose fibers) used for hydroentanglement may have a median length of 5 to 30 mm, preferably from 10 to 20 mm. Fiber length can be measured using a microscope with a scale. An arithmetic average may be calculated from the lengths of a multitude of fibers of a representative sample of fibers. The median length of the fibers may be determined from the lengths of fibers of a representative sample of fibers.

The top sheet may have perforations allowing body exudate to be quickly transferred to the ADL and further to the absorbent layer. The perforations may have a size of from 0.2 to 10 mm$^2$, preferably of from 0.5 to 5 mm$^2$. The perforations may be preferably located in an area of the absorbent product, that contacts body parts from which body exudate is to be expected.

The top sheet may be at least partially coated with, and may thus contain a layer of, a hydrophilic polymer, preferably at least on its outer surface facing the body of the user. Examples of hydrophilic polymers are carboxymethyl cellulose, alginates or other hydrogels like gelatin, chitosan, or hyaluronic acid or a salt of the afore-mentioned polymers.

The basis weight of the top sheet nonwoven may be from 20 to 50, preferably 30 to 40 g/m$^2$. Its average density may be from 80 to 160, preferably from 100 to 140 kg/m$^3$. The wicking rate of this top sheet material may be 0 measured by EDANA ERT 10.3-99. The thickness of the top sheet is generally within a range of from 0.1 to 4 mm, preferably from 0.2 to 2 mm.

Acquisition and distribution layer (ADL)

ADLs in absorbent products were introduced to provide an intermediate storage for body exudate, providing more time for absorbent cores (mostly those containing superabsorbents) to slowly absorb the exudate which already have been wetted in use. ADLs of prior art products are typically made of thick, bulky, and resilient nonwovens. In many products, the nonwovens are made of polyester fibers, as they offer better stiffness and regain their original volume when pressure applied to them is released. ADLs generally allow for spreading the fluid within the plane of the layer by the wicking ability of the ADL, as well as in the thickness (z) direction of the ADL. The thickness and resilience are not that important for ADLs. Thus, even thin, well compressed fibrous layers can provide a good wicking and distributing performance.

The ADL of the product of the invention may consist of one layer providing the acquisition and distribution function, or may comprise two or more layers, one providing mainly the acquisition and another one providing mainly the distribution function. Preferably, the ADL is a sheet in the sense of the invention, i.e. the ADL is a sheet made of polysaccharide. The ADL should consist of polysaccharide, preferably cellulose or a cellulose derivative (such as cross-linked cellulose). Said ADL may be or may comprise a fibrous batt. A batt is a fibrous structure that is a loose assemblage of fibers.

The ADL of the absorbent product of the invention generally is or comprises a fibrous batt, preferably a nonwoven fabric made of cellulose material or made of material of a cellulose derivative, as for example crosslinked, stiffened and curled cellulose fibers, and viscose fibers. The ADL may be a sheet made of such nonwoven. Preferably, the nonwoven is made of crosslinked cellulose or of a mixture of cellulose and crosslinked cellulose. The nonwoven may be made of cellulose fibers and/or of fibers of a cellulose derivative, preferably made of cross-linked cellulose fibers. The fibers may have, before cross-linking, a median length of 2 mm or longer, preferably a median length of from 2.0 to 15 mm. In the process of crosslinking, hydroxyl groups of a D-glucose unit of one cellulose molecule will be covalently bonded to a hydroxyl group of another D-glucose unit of the same or an adjacent cellulose molecule.

In one embodiment, the ADL is or comprises at least one sheet made of cellulose material, preferably made of cellulose fibers, said fabric preferably having a density of from 0.10 to 0.40 g/cm$^3$, preferably of from 0.20 g/cm$^3$ to 0.35 g/cm$^3$ and being a sheet of (bathroom) tissue paper.

In another, preferred, embodiment, the ADL comprises at least two elements: a sheet of tissue paper and a layer of crosslinked cellulosic fibers which may be laid down in an air stream directly on the tissue layer or constitute a separate web of a fabric. While the crosslinked cellulosic fibers provide volume and resilience to the ADL and thus offer in a z direction a void volume for a fast intake of body exudates, the tissue paper provides a mechanical strength necessary for the use of such material in a production line of absorbent hygienic products as well as good wicking resulting in a good distribution of the fluid in x and y direction.

The density of the ADL layer consisting of crosslinked fibers is generally lower than the density of the top sheet, but the density of a tissue sheet or similar nonwoven fabric made of cellulose fibers is generally higher than the density of the top sheet, which results in an improved transport of the fluid toward the material of higher density as its capillaries are smaller. The wicking ability of the distribution layer is significantly higher than the wicking ability of the top sheet, e.g. due to the perforation of the top sheet.

The ADL layer and/or said nonwoven may have a wicking rate with synthetic urine at least two times higher than the wicking rate of the top layer. This supports transfer of body fluid away from the top sheet to the ADL. The wicking rate may be measured as described in Part 6 go ISO 9073.

The ADL and/or the nonwoven fabric used for the ADL preferably has a low specific water absorption capacity. The specific water absorption may be in the range of from 1.5 g/g to 3.0 g/g. Water absorption capacity and water absorption time can be determined by the International test method ISO 20158:2018 which is normally used for textiles.

In a preferred embodiment, the ADL is a sheet of one or more layers of cellulose or a cellulose-derivative

For the comfort of a user, an important issue is how much fluid can be - even under pressure - irreversibly absorbed by a product. For functional layers close to the skin as a material with a lot of moisture will expose the skin to a wetness for a long time, which is not only uncomfortable but also may result in skin damage. Therefore, at least one or more of the sheets of the ADL should be hydrophilic, but should not absorb fluids in large amounts, as the void volume of the ADL should remain available not only for the first, but also for subsequent discharges of body fluid to the product. A possible way to provide such property to the sheet of the ADL used in the present invention, is, as indicated above, the use of crosslinked cellulose or crosslinked cellulose derivatives as the material from which the sheets are made.

The ability to retain fluids under pressure is known as retention and can be measured according to ASTM D 2402-01 "Standard Test Method for Water Retention of Textile Fibers (Centrifuge Procedure). The retention of the ADL and/or a sheet of the ADL is preferably in the range of from 0.5 to 1.0 g/g.

To ensure good fluid flow from the top sheet to the ADL, the top sheet and the ADL may be mechanically bonded with each other e.g. by embossing points and lines producing a narrow gap between both layers. Thus, after release of fluid from the user, the wet area of the top sheet will be relatively small as the fluid will flow fast to the ADL where it distributes to a larger area.

Absorbent layer

The absorbent layer serves to receive, store and to some extent bind irreversibly body fluids. The absorbent layer is sometimes also referred to in the art as absorbent core. Typical absorbent cores of the prior art comprise fibers and particles of superabsorbent polymers. While fibers provide fluid distribution and a certain absorbent core integrity, superabsorbent particles absorb and store the fluid. Performance parameters of absorbent cores include speed of absorption, absorption capacity, and fluid retention.

The absorbent layer of the absorbent product of the invention generally comprises a superabsorbent material, preferably superabsorbent material of a cellulose derivative. The absorbent layer may be of several general types: a first, simpler, type wherein the absorbent layer consists of superabsorbent material; in the second type, the absorbent layer comprises one or more (generally non-superabsorbent) sheets and a superabsorbent that is distributed on one or more sheets or the superabsorbent is enclosed on one or two sides by said sheet(s), or the superabsorbent is mixed with fibers that together constitute a layer within an absorbent core. In a third type, no superabsorbent is present in the absorbent layer.

The superabsorbent is generally in the form of particles. The, preferably particulate, superabsorbent may or may not be a polysaccharide, or may not exclusively consist of superabsorbent polysaccharide. Instead, the superabsorbent may be or may comprise non-biodegradable superabsorbent, such as polyacrylic acid or a salt thereof, preferably particles of polyacrylic acid or a salt thereof. However, it is preferred that the superabsorbent consists of (biodegradable) polysaccharide, such as polysaccharide particles, more preferably particles of a cellulose derivative such as particles of CMC. CMC is commercially available from Evonik, BASF, Nippon Shokubai, or Sanyo.

In a general embodiment of the second type, the absorbent layer comprises one or more sheets and superabsorbent material within or on said one or more sheets, said sheets being made of cellulose material and/or of material of a cellulose derivative, such as of fibers of these materials. Preferably, the absorbent layer comprises two or more sheets enclosing a superabsorbent material between said sheets, said sheets may be made of cellulose material and/or of a material of a cellulose derivative, preferably cellulose.

In one embodiment of the general embodiment, said one or more sheets may be made of dry defiberized cellulose, such as fluff, and/or may be made of tissue paper of cellulose. The one or more sheets may be obtainable from dry defiberized cellulose pulp, e.g. by laying down the pulp by an air stream and subsequently compressing to a density of at least 100 kg/m$^3$. The preferred embodiments of the sheets of the absorbent layer can be combined with the preferred embodiments of the superabsorbent.

In an alternative embodiment of the general embodiment, the absorbent layer comprises one or more sheets of nonwoven made of cellulose fibers and/or of fibers of a cellulose derivative (the former being preferred) and particles of a superabsorbent attached to said nonwoven fabric sheet. The superabsorbent is as described above.

In a further alternative embodiment of the general embodiment, the absorbent layer comprising at least two sheets of cellulose porous material and/or of material of a cellulose derivative (the former being preferred), such as tissue paper or wet-laid or spun-laced nonwoven, and superabsorbent particles between said layers. The superabsorbent is as described above.

In the second type, the sheets enclosing the superabsorbent may be connected to form compartments in order to avoid that the superabsorbent flows over large distances within the absorbent layer, but remains within such compartments.

An embodiment of the third type is as follows. The absorbent layer comprises fibrous cellulose material (fluff) encased by tissue layers and compressed to a density of 120 to 160, preferably 130 to 150 kg/m$^3$. The basis weight is 170 to 230, preferably 190 to 210 g/m$^2$. This layer can be additionally embossed, e.g., with a diamond pattern to produce spot wise a very high density and improve the mechanical properties important in manufacturing process.

The absorbent layer and/or the sheet(s) has, at first wetting, a wicking rate with synthetic urine of at least two times the wicking rate of the top layer. As synthetic urine, a 0.9% NaCl (analytical grade) solution in de-ionized water, conductivity at 25° C of 16 mS can be used according to EDANA Test Methods.

Back sheet

The back sheet together with top sheet constitute an envelope of the absorbent product of the invention. The role of the back sheet is to provide a containment for fluids absorbed by the product and to prevent the wetted product from leaking and staining the wearer's clothes.

The back sheet is or comprises a sheet of cellulose material or material of a cellulose derivative. The back sheet may be or may comprise a cellulose film such as a cellophane film. The back sheet may contain or may be coated with a softening agent such as glycerol.

In one embodiment, the back sheet comprises or consists of a cellulose film or a film of a cellulose derivative (the former being preferred), laminated with a nonwoven made of cellulose fibers, wherein said film laminated with a nonwoven preferably has a basis weight of 5 to 40 g/m$^2$, preferably 10 to 30 g/m$^2$, and even more preferably 15 to 25 g/m$^2$.

Alternatively, the back sheet may be a sheet made of or comprising cellulose fibrous material, said sheet having pores with a pore size not large enough for water droplet transmission, but large enough for water vapor transmission. Thus, the layer may be breathable. The pore size of said sheet may be smaller than 100 μm, preferably smaller than 50 μm. In one embodiment, the back sheet does not have relevant pores.

The absorbent product has, as explained above, a top sheet that is fluid permeable and may for that purpose, have pores. On the other hand, the back sheet is fluid impermeable. If the back sheet has pores, the median pores size of pores of the top sheet is at least 20 times, preferably at least 50 times, more preferably at least 100 times larger than the median pore side of pores in the back sheet. This embodiment covers the case wherein the back sheet does not have relevant pores.

To generate a suitable soft, pleasant feel of the back sheet, plasticizers can be added, such as glycerol. Antioxidants may also be included in the back sheet or film. Desired opacity of the back sheet can be achieved by addition of neutral, mineral fillers, such as talc or silica.

Such technology is described in "The Plasticization Effect of Glycerol and Water on the gelatinization of Wheat Starch by Gena Nashed et al. (April 2003, Starch - Starke 55 (3-4), DOI: 10.1002/star.200390027). Another way to obtain such films is described in "Novel Starch/Chitosan/Aloe Vera Composites as Promising Biopackaging Materials" by Dagmara Baier et al., J. Polym. Environ 28, 1021-1039 (2020). Xu et al. describe in "Robust and Flexible Films from 100% Starch Cross-Linked by Biobased Disaccharide Derivative" the use of oxidized sucrose for crosslinking the starch (ACS Sustainable Chem. Eng. 2015, 3, 11, 2631-2639). Ning et al. describe the advantage of using citric acid in "Influence of Citric Acid on the Properties of Glycerol-Plasticized Cornstarch Extrusion Blends" (School of Science, Tianjin University, China, in Polymers & Polymer Composites, Vol. 15, No. 7, 2007).

A fibrous sheet as a back sheet can be produced by wet laid technology in which a suspension of fibers in water will be dewatered on a screen, whereby a fibrous web is formed and remains for further process. Papers, consisting of cellulose pulp fibers, are typically produced by such process. Wet-laid nonwovens can use other, longer fibers, e.g., fibers made of regenerated cellulose like viscose.

Depending on the manufacturing process such nonwovens can be wet-laid or airlaid made. A good description of wet-laid cellulosic nonwovens is given by Hemamalini in "Wet laying nonwovens using natural cellulosic fibers and their blends: process and technical applications. A review" (https://doi.org/10.1080/15440478.2019.1701606). Dry laid nonwovens made of cellulosic fibers are known to those skilled in the art. As an example, dry or air laid nonwovens made of cotton or viscose fibers by carding or Rando process are available in the market.

A modern viscose-based nonwoven with reduced binder content is described in EP 2138056 A3. A new approach to a viscose-based nonwoven is described in CN2012100077076A. This application covers a spunlaid viscose nonwoven which can be tight enough to provide a barrier function to fluids and a good permeability to water vapor.

A back sheet made of cellulosic fibers and best suitable for the use in this invention has a good breathability, will be non-permeable to fluids like urine and blood under in-use conditions and will preferably be hydrophobic on its inner (facing absorbent core) side.

The back sheet is preferably hydrophobic in nature, as the back sheet should provide a barrier to fluids and forms a container for them. Thus, the back sheet may be coated to provide a desired level of hydrophobicity. A person skilled in the art will appreciate several bio-materials which can be used as a treatment or coating to a wet-laid material and provide a hydrophobicity to it. A method that uses starch as a hydrophobicity (repellency) agent is described in WO2018050317.

Another requirement for a back sheet in absorbent hygienic products is softness which provides a pleasant feel for a use and lack of noise when a user moves. Both properties can be improved using a fiber mix containing at least several percent of short cellulosic fibers as well as a mechanical treatment to the sheet. Such treatment can comprise embossing, corrugating and similar deformations of plain web surface. As an example, a soft embossing of a fibrous structure is described in US 6,468,392. While this patent describes mostly hydrophilic fibrous structures, a combination with a hydrophobisation step will result in desired properties for a back sheet. Also, a use of socalled air-through dryer prior to the hydrophobisation step can improve the softness and "silence" of such web. A paper machine equipped with such air-through-dryer is described in US6398916.

This invention also relates to back sheets made of poly(butylene succinate cobutylene adipate) known as PBSA polymer which can be obtained from renewable feedstock such as glucose and sucrose. Also, thermoplastic starch (TPS) enhanced by additives and plasticizers can be used for producing a back sheet. A further example of a material usable for producing a back sheet is polyhydroxyalkanoates (PHA).

_Adsorbent product_

A product according to this invention can be manufactured on machines known and present in the industry as sanitary napkin machines or similar production lines for diapers, panty shields or light incontinence pads. The manufacturing process comprises subsequent steps of putting individual product components together which can be described as assembling. The elements can be cut to desired shape and dimension. The chemical composition of product elements does not change during the manufacturing process, so the properties - as an example the biodegradability - of a component remain unchanged in a final product. A typical machine of this art provides an absorbent core forming unit (cellulose web unwind, defiberizing mill, lay-down unit, superabsorber dosing unit) or an unwind for a pre-made absorbent core composite, several unwinds for further components as top sheet, ADL, core wrap, back sheet etc. The webs of these materials may be subsequently positioned on a transportation belt. First the back sheet, followed by wrapped absorbent core, followed by ADL, and then be covered by the top sheet. The sequence can be reversed and the product may be manufactured top-down which is advantageous when the back sheet is not air-permeable while an air-permeable top sheet can be easily held by vacuum on the transportation belt of the machine. Layers may be cut to proper length and/or shape before the entire product after its assembly will be end-cut to the final length, folded and individually wrapped. The layers may be connected to each other by means of glue (hotmelt), pressure, etc. The glue may also be biodegradable, e.g. may comprise natural or nature-similar resins. Usable machines are available from machine producers like Zuiko (Japan), Curt Joa (USA), Winkler+Dünnebier (Germany) or Fameccanica (Italy).

In the absorbent product of the invention, at least two of said at least four layers may be bound to each other e.g. by mechanical embossing or stitching, preferably at least along the periphery of said product so that the product forms a bag-like structure enclosing the absorbent layer. Compartments may be formed within the flat plane of the product, e.g. by embossing or stitching to areas from which superabsorbent cannot move away.

The absorbent product of the invention can - for the discretion and the comfort of the wearer - be individually packaged. Accordingly, the invention provides a packaged absorbent product comprising the absorbent product wrapped in a film made of cellulose material or a material of a cellulose derivative, such as cellophane, or wrapped in a sheet made of cellulose fibers.

The absorbent product of the invention may be an absorbent hygiene product such as a sanitary napkin, diaper, or incontinence pad. In another embodiment, the product is a wound dressing. For the latter, the individual packaging can ensure the cleanliness and for sterilized dressings the sterility before use.

EXAMPLES

Example 1 (Reference Example)

A "night" sanitary napkin / incontinence pad comprising polypropylene/polyethylene bicomponent carded high-loft nonwoven as a top sheet

Acquisition and Distribution Layer (ADL) made of crosslinked cellulose fibers
Absorbent Core consisting of a composite material comprising cellulose fibers mixed with a polyacrylate-based superabsorbent polymer
Back sheet made of a polyethylene film.

Such product comprises some elements based on natural resources like crosslinked cellulose fibers and fluff fibers in its absorbent core but the majority of the constituents of the product is based on fossil resources, thus not renewable and not biodegradable.

**Example 2** (Reference Example)

A sanitary napkin / light incontinence pad comprising cotton nonwoven top sheet of the top layer;
Cotton fiber pad, as a first layer below the top sheet, in-part functioning as an ADL; Cellulosic composite material positioned below the cotton fiber pad; and Back sheet made of a Mater-Bi film.
Such product is largely biodegradable and mainly bio-material based. The back sheet, however, consists of a plastic material which even if containing a high percentage of starch still does not fulfill the exclusion criteria of SUP directive.

**Example 3**

A sanitary napkin / light incontinence pad comprising:

Cotton nonwoven as a top sheet
Cotton fiber pad as a first layer below the top sheet (ADL)
Cellulosic composite material positioned below the cotton fiber pad; and
Back sheet made of a transparent, regular "Cellophane" regenerated cellulosic film.

Such product is biodegradable and fully bio-material based. It also fulfills the SUP directive criteria to be not categorized as plastic. Its back sheet, however, consists of a material which is relatively stiff, crackle and rustle, which makes it "loud" and not so pleasant for the user, as the sound can be noticed by people in the vicinity of the user. Also, the transparent back sheet does not mask the body fluids absorbed by the hygiene product which can be irritating to the user.

**Example 4**

A sanitary napkin / light incontinence pad comprising:

cotton nonwoven as a top sheet;
an ADL made of cross-linked cellulose fibers as a first layer below the top sheet;
a cellulose composite material layer (absorbent layer) positioned below the crosslinked cellulose fiber pad; and
a back sheet made of a transparent cellophane regenerated cellulose film which has been softened by means of addition of softening agents like glycerol and was embossed to significantly reduce the crackling noise. The cellophane film has a reduced transparency which results in reduced transparency and significantly reduced visibility of stain from body effluents. Also, a cellophane film used as a back sheet improves a cross-direction stability of the product when worn in the crotch area of the wearer and thus exposed to compression between the legs.

Such product is biodegradable and fully bio-material based. It also fulfills the SUP directive criteria to be not categorized as plastic. The cellulose film offers properties similar to standard plastic films e.g., low crackling level and good opacity.

**Example 5**

An absorbent hygiene product as described in Example 4, wherein the cellulose composite material of the absorbent layer contains superabsorbent particles, said superabsorbent being of a bio-based type and based on cellulose. The amount of said superabsorbent in said composite material is between 10 and 80 %, preferably between 30 and 60%, and most preferably between 40 and 55% by weight.

**Example 6**

A sanitary napkin / light incontinence pad comprising:

cotton nonwoven as a top sheet, said top-sheet being spun-laced and showing perforations spread over its entire surface, said top sheet showing zero or a minimal wicking ability to body fluid;
an ADL made of cross-linked cellulose fibers as a first layer below the top sheet, said layer providing a rapid transfer of the fluid towards the absorbent core below it;
absorbent layer of cellulose composite material positioned below the cross-linked cellulose fiber pad and consisting of cellulose fibers mixed with a cellulose-based superabsorbent polymer, said superabsorbent polymer showing a slow absorbency and allowing the fluid entering the fibrous structure to wick along the absorbent core layer; and

a back sheet made of a fibrous structure comprising cellulose fibers laid down as a wet-laid nonwoven and being not permeable to fluid but breathable.

Single wrapping (individual packaging) consisting of a cellophane regenerated cellulosic film which has been softened by means of addition of softening agents like glycerol and was embossed to significantly reduce the crackling noise. The cellophane film has a reduced transparency which results in significantly lower visibility of stain from body effluents in post-use products.

**Example 7**

A wound dressing consisting of a top sheet being the wound contact layer, said top sheet being made of a nonwoven fabric comprising a majority of cellulose fibers, preferably viscose, treated with alginates to ensure non-sticking to the wound. The top sheet has perforations allowing wound exudate to be easily transferred to the inner layers. The inner layer consists of a matrix made of cellulose fibers (ADL) and superabsorbent particles based on cellulose. The back sheet made of transparent cellophane allows for rapid visual inspection of the dressing and the wound's vicinity.

**Test methods**

Density

Density is the mass per unit volume of a material. Density and specific gravity of the material can be measured according to ASTM D792 and ISO 1183 standardized test methods.

Basic weight

Basic weight is the weight of a surface unit of a product. It can be measured based on ASTM D646-96, reapproved 2001.

Transparency / opacity

The transparency of materials can be measured based on ASTM D1746-15

Stiffness

The stiffness of materials can be measured by so called Taber Method according to ASTM D5650 / TAPPI T566

Cross Direction Resilience

This parameter can be measured by estimation of so called Gurley Stiffness. Gurley stiffness measures the bendability or flexibility of absorbent materials. The lower the Gurley stiffness value, the more flexible the material. The Gurley stiffness values are measured using a Gurley Stiffness Tester (Model No. 4171 E), manufactured by Gurley Precision Instruments of Troy, N.Y. The instrument measures the externally applied moment required to produce a given deflection of a test strip of specific dimensions fixed at one end and having a concentrated load applied to the other end. The results are obtained in "Gurley Stiffness" values in units of milligrams.

Wicking / Fluid distribution

Wicking action can be measured based on ISO 9073 which among other properties describes Liquid Wicking Rate being a measure of the capillarity action of the test material, i.e. the rate at which the liquid is transported into the fabric by capillary action.

Pore size (Quote from EP 1074234)

A useful method to measure the average pore size of a nonwoven material works with liquid extrusion cell, developed at Textile Research Institute, Princeton, New Jersey, USA. This technique has been described in Miller et al., "An Extended Range Liquid Extrusion Method for Determining Pore Size Distributions", Textile Research Journal, Vol. 56, pp. 35-40 (1986), herein incorporated by reference, and it was used to derive a mathematical model to predict the average pore size of a nonwoven fabric, Cohen, "A Wet Pore-Size Model for Coverstock Fabrics", Book of Papers: The International Nonwoven Fabrics Conference. INDA-TEC'90, Association of the Nonwoven Fabrics Industry, pp. 317-330 (1990), herein incorporated by reference. Based on this model, the following equation was used in the determination of average pore sizes reported in the specification:

$$r = (\Sigma_i x_i a^2 / \Sigma_i x_i a) \ ((\rho_f / \xi \rho_w) - 1) / \tau$$

wherein

r is the average pore radius; a is the fiber radius; x is a number fraction;
$\xi$ is the ratio of dry fabric density to wet fabric density; pf is the fiber density; pw is the dry fabric density; and
$\tau$ is the tortuosity parameter.

**Claims**

1. An absorbent product having a sequence of layers and comprising at least the following layers in this order:

   (i) a fluid-permeable top sheet facing the body of the user,
   (ii) an acquisition and distribution layer (ADL),

(iii) an absorbent layer, and
(iv) a fluid-impermeable back sheet,

wherein at least layers (i) and (iv) are or comprise a sheet made of a polysaccharide, preferably made of cellulose or a cellulose derivative.

2. The absorbent product according to claim 1, wherein layers (i), (ii), and (iv) comprise one or more sheets made of cellulose or a cellulose derivative; or wherein each of said four layers (i) to (iv) comprises a sheet consisting of or made of a cellulose material or of a material of a cellulose derivative as the material forming said sheets, preferably of cellulose.

3. The absorbent product according to claim 1 or 2, wherein said top sheet is or comprises a sheet that is a fabric made of cellulose fibers, such as of cotton and/or viscose fibers, preferably said fabric is a nonwoven fabric that may be produced by a method comprising hydroentanglement (spunlacing).

4. The absorbent product according to any one of claims 1 to 3, wherein said ADL is or comprises a fibrous batt made of cellulose or a cellulose derivative, or is a nonwoven fabric made of cellulose or a cellulose derivative.

5. The absorbent product according to claim 4, wherein said ADL is or comprises a nonwoven fabric made of cellulose fibers and/or of fibers of a cellulose derivative, preferably made of cross-linked cellulose fibers, said fabric preferably having a density of 0,20 g/cm$^3$ or lower.

6. The absorbent product according to any one of claims 1 to 5, wherein said absorbent layer comprises a superabsorbent, preferably the superabsorbent comprises or consists of a cellulose derivative.

7. The absorbent product according to any one of claims 1 to 5, wherein said absorbent layer comprises one or more sheets and a superabsorbent within said one or more sheets, said sheets being made of cellulose and/or of a cellulose derivative.

8. The absorbent product according to any one of claims 1 to 7, wherein said absorbent layer comprises two or more sheets enclosing a superabsorbent between said sheets, said sheets being made of cellulose and/or of a cellulose derivative, such as cellulose fibers.

9. The absorbent product according to any one of claims 6 to 8, said absorbent layer comprising a nonwoven fabric sheet made of cellulose fibers and/or of fibers of a cellulose derivative, and particles of a superabsorbent attached to said nonwoven fabric sheet, said superabsorbent preferably comprising or consisting of a cellulose derivative.

10. The absorbent product according to any one of claims 1 to 8, said absorbent layer comprising at least two sheets of porous cellulose material, such as tissue paper or wet-laid or spun-laced nonwoven, and superabsorbent polymer particles between said layers.

11. The absorbent product according to any one of claims 6 to 10, wherein said superabsorbent is or comprises carboxymethyl cellulose (CMC) and/or a polyacrylic acid, or salts thereof, preferably said superabsorbent consists of CMC or a salt thereof, more preferably particles of CMC.

12. The absorbent product according to any one of claims 1 to 11, wherein said back sheet is or comprises a sheet or film of cellulose material or material of a cellulose derivative, preferably said back sheet is a cellulose film such as a cellophane film; said back sheet may contain or may be coated with a softening agent such as glycerol.

13. The absorbent product according to any one of claims 1 to 12, wherein said back sheet comprises cellulose fibrous material, said sheet having pores with a pore size not large enough for water droplet transmission but large enough for water vapor transmission, thus being breathable; preferably, the pore size of said sheet being smaller than 100 $\mu$m, preferably smaller than 50 $\mu$m.

14. The absorbent product according to any one of claims 1 to 13, which is an absorbent hygiene product such as a panty shield, sanitary napkin, a diaper, or an incontinence pad, or is a wound dressing.

15. A packaged absorbent product comprising the absorbent product according to any one of claims 1 to 14 wrapped in a film made of cellulose material or material of a cellulose derivative, such as cellophane, or wrapped in a sheet made of cellulose fibers.

Fig. 1

Europäisches
Patentamt

European
Patent Office

Office européen
des brevets

# EUROPEAN SEARCH REPORT

**Application Number**

EP 22 19 2219

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GB 2 492 171 A (SCA HYGIENE PROD AB [SE]) 26 December 2012 (2012-12-26) | 1-14 | INV. A61F13/00 |
| Y | * page 4, line 4 - page 6, line 15 * ----- | 15 | A61F13/15 A61F13/511 |
| X | EP 3 895 673 A1 (ONTEX BV [BE]; ONTEX GROUP NV [BE]) 20 October 2021 (2021-10-20) | 1-14 | A61F13/513 A61F13/514 |
| Y | * paragraph [0064] * * paragraph [0072] - paragraph [0075] * * paragraph [0080] - paragraph [0082]; figure 3 * ----- | 15 | |
| X | US 2017/056253 A1 (CHESTER STEPHEN O [US] ET AL) 2 March 2017 (2017-03-02) | 1-14 | |
| Y | * paragraph [0040] - paragraph [0074] * * paragraph [0118] - paragraph [0122]; figures 1-4 * ----- | 15 | |
| Y | US 2021/298961 A1 (LIU SHENG [NZ]) 30 September 2021 (2021-09-30) * paragraph [0068] - paragraph [0072] * ----- | 15 | |
| A | US 5 300 358 A (EVERS GLENN R [US]) 5 April 1994 (1994-04-05) * example 1 * ----- | 1-15 | **TECHNICAL FIELDS SEARCHED (IPC)** A61F |

The present search report has been drawn up for all claims

| Place of search | Date of completion of the search | Examiner |
|---|---|---|
| Munich | 13 January 2023 | Demay, Stéphane |

EPO FORM 1503 03.82 (P04C01)

**EP 4 327 790 A1**

## ANNEX TO THE EUROPEAN SEARCH REPORT
## ON EUROPEAN PATENT APPLICATION NO.

EP 22 19 2219

This annex lists the patent family members relating to the patent documents cited in the above-mentioned European search report.
The members are as contained in the European Patent Office EDP file on
The European Patent Office is in no way liable for these particulars which are merely given for the purpose of information.

13-01-2023

| Patent document cited in search report | | Publication date | Patent family member(s) | | Publication date |
|---|---|---|---|---|---|
| GB 2492171 | A | 26-12-2012 | NONE | | |
| EP 3895673 | A1 | 20-10-2021 | EP | 3895673 A1 | 20-10-2021 |
| | | | WO | 2021209384 A1 | 21-10-2021 |
| US 2017056253 | A1 | 02-03-2017 | CN | 108472175 A | 31-08-2018 |
| | | | EP | 3340953 A1 | 04-07-2018 |
| | | | JP | 2018525170 A | 06-09-2018 |
| | | | PE | 20180817 A1 | 09-05-2018 |
| | | | US | 2017056253 A1 | 02-03-2017 |
| | | | WO | 2017037550 A1 | 09-03-2017 |
| US 2021298961 | A1 | 30-09-2021 | AU | 2021201952 A1 | 14-10-2021 |
| | | | US | 2021298961 A1 | 30-09-2021 |
| | | | WO | 2021201698 A1 | 07-10-2021 |
| US 5300358 | A | 05-04-1994 | NONE | | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 520576 A
- GB 373758 A
- GB 549254 A
- US 2024976 A
- GB 362046 A
- GB 430269 A
- US 10105269 B
- US 9579238 B
- US 2019201252 A1
- EP 0878204 A2
- EP 1435247 A1
- EP 0405993 A
- US 6169223 B, Mahr
- EP 91905693 B1
- US 3489148 A

- US 8530722 B
- EP 2399558 A
- EP 1369519 A
- EP 2708623 A
- WO 2014022988 A
- EP 3246444 A
- DE 3312022 A1
- DE 3246417 A1
- EP 2138056 A3
- CN 2012100077076 A
- WO 2018050317 A
- US 6468392 B
- US 6398916 B
- EP 1074234 A

**Non-patent literature cited in the description**

- **J.R. ELLIOTT.** The Development of Cotton Wool as a Wound Dressing. *Med. Hist.,* October 1957, vol. 1 (4), 362-366
- **E. PINHO ; G. SOARES.** Functionalization of cotton cellulose for improved wound healing. *Journal of Materials Chemistry B,* 2018, (13
- **ABAZARI et al.** Recent advances in cellulose-based structures as the wound-healing biomaterials. A clinically oriented review. *Appl. Sci.,* 2021, vol. 11, 7769
- **WILD et al.** Wound cleansing efficacy of two cellulose-based dressings. *Wounds UK,* 2010, vol. 6 (3
- Carboxymethyl cellulose-based materials for infection control and wound healing: A review. *International Journal of Biological Macromolecules,* 01 December 2020, vol. 164, 963-975
- **TOMILLA et al.** *Cellulose - a biomaterial with cell-guiding property, https://www.intechopen.com/chapters/45634*
- **GENA NASHED et al.** The Plasticization Effect of Glycerol and Water on the gelatinization of Wheat Starch. *Starch - Starke,* April 2003, vol. 55 (3-4

- **DAGMARA BAIER et al.** Novel Starch/Chitosan/Aloe Vera Composites as Promising Biopackaging Materials. *J. Polym. Environ,* 2020, vol. 28, 1021-1039
- **XU et al.** Robust and Flexible Films from 100% Starch Cross-Linked by Biobased Disaccharide Derivative. *ACS Sustainable Chem. Eng.,* 2015, vol. 3 (11), 2631-2639
- Influence of Citric Acid on the Properties of Glycerol-Plasticized Cornstarch Extrusion Blends. **NING et al.** Polymers & Polymer Composites. School of Science, Tianjin University, 2007, vol. 15
- **MILLER et al.** An Extended Range Liquid Extrusion Method for Determining Pore Size Distributions. *Textile Research Journal,* 1986, vol. 56, 35-40
- A Wet Pore-Size Model for Coverstock Fabrics. **COHEN.** Book of Papers: The International Nonwoven Fabrics Conference. INDA-TEC'90. Association of the Nonwoven Fabrics Industry, 1990, 317-330